# EUROPÄISCHE PATENTSCHRIFT

(11) **EP 3 393 388 B1**
(45) Veröffentlichungstag und Bekanntmachung des Hinweises auf die Patenterteilung: **26.06.2019**
(21) Anmeldenummer: 18710827.9
(22) Anmeldetag: 08.03.2018
(51) Int. Cl.: A61B 50/30, A61B 90/00, A61B 50/00

(54) **BEHÄLTER ZUM STERILEN VERPACKEN VON GEGENSTÄNDEN UNTER REDUZIERTEM DRUCK**
CONTAINER FOR STERILE PACKAGING OF ITEMS UNDER REDUCED PRESSURE
CONTENEUR POUR L'EMBALLAGE STÉRILE D'ARTICLES SOUS PRESSION RÉDUITE

(30) Priorität: 16.03.2017 DE 102017105659
(43) Veröffentlichungstag der Anmeldung: 31.10.2018
(73) Patentinhaber: Linhardt GmbH & Co. KG, 94234 Viechtach (DE)
(72) Erfinder: BEIL, Johann, 93462 Lam (DE)
(74) Vertreter: Glück Kritzenberger Patentanwälte PartGmbB
(86) Internationale Anmeldenummer: PCT/EP2018/055791
(87) Internationale Veröffentlichungsnummer: WO 2018/166892

(56) Entgegenhaltungen:
- CH-A- 453 927
- DE-A1- 10 146 905
- DE-C1- 19 908 845
- DE-U- 1 723 232

## Beschreibung

### Technisches Gebiet

Die Erfindung bezieht sich auf einen Behälter zum sterilen Verpacken von Gegenständen unter reduziertem Druck.

### Stand der Technik

Behälter zum Verpacken und Aufbewahren von Gegenständen unter sterilen Bedingungen sind aus dem Stand der Technik bekannt. Häufig werden die sterilen Bedingungen über einen längeren Zeitraum dadurch aufrecht erhalten, dass die Gegenstände unter reduziertem Druck aufbewahrt werden.

In diesem Zusammenhang offenbart die US 4,149,650 einen Aufbewahrungsbehälter für Gegenstände und Werkzeuge, die unter sterilen Bedingungen gelagert werden müssen. Der Behälter umfasst ein Unterteil sowie einen Deckel mit einem Dichtungsstreifen. Der Behälter wird unter Vakuum befüllt, wodurch das Unterteil und der Deckel nachfolgend aufgrund des äußeren Luftdrucks zusammengehalten werden. Auf der Oberseite des Deckels ist ein Lüftungsventil angebracht, mit dessen Hilfe das Vakuum im Inneren des Behälters aufgehoben werden kann, sodass sich der Behälter öffnen lässt und die darin befindlichen Werkzeuge bzw. Instrumente entnommen werden können. Der Dichtungsstreifen besteht aus einem wärmeschrumpfenden Kunststoff, der über das Ventil aufgeschrumpft wird.

Die DE 33 29 941 A1 offenbart einen mehrteilig ausgebildeten Behälter zum Verpacken bzw. Einlagern von Lebensmitteln. Der Behälter ist mit einem Ventil ausgestattet, durch das mittels einer Vakuumpumpe ein Vakuum angelegt werden kann. Über dem Ventil ist eine Abreißfolie angeordnet, die zum Aufheben des Vakuums abgenommen werden kann. Der Behälter weißt zwei umlaufende Dichtelemente auf, die im Querschnitt ein H-förmiges Profil zeigen.

Die US 2016/0106507 A1 betrifft eine Verpackung für medizinische Gegenstände, vorzugsweise medizinische Implantate, bestehend aus zwei über ein Scharnier verbundene, aufeinander zu liegend kommende Schalen. Bei geschlossenem Zustand ist über die gesamte Länge der Ränder der Schalen ein Dichtungsstreifen angebracht. Der Dichtungsstreifen dient dazu, die beiden Schalen abzudichten, sodass der Verpackungsinhalt in sterilem Zustand gelagert bzw. transportiert werden kann. Es sind keine weiteren Verschlusselemente zum Verschließen des Behälters vorgesehen. Zum Öffnen des Behälters wird der aufgeschrumpfte Dichtungsstreifen entfernt und der darin befindliche Gegenstand kann entnommen werden.

Die Druckschriften DE 10 2006 034 527 A1 und DE 26 51 291 A1 zeigen mehrteilig ausgebildete Sterilbehälter, umfassend ein Oberteil, ein Unterteil, einen Vakuumverschluss bzw. eine Vakuumverriegelung sowie Dichtelemente. Bei der DE 10 2006 034 527 A1 erfolgt die Verriegelung des Deckels auf dem Unterteil während des Sterilisationsvorgangs. Mechanische Verriegelungen sind nicht notwendig, da durch das erzeugte Vakuum innerhalb des Sterilbehälters die beiden Behälterhälften fest miteinander verschlossen werden. Bei der DE 26 51 291 A1 wird während eines Sterilisationsvorgangs, z.B. in einem Autoklaven, an einen Behälter Vakuum angelegt. Der Behälter umfasst ein Einwegventil, das sich gegen Ende des Sterilisationsvorgangs schließt. Das Ventil wird durch ein erweichendes bzw. schmelzendes Verschlusselement so lange offen gehalten, bis eine ausreichend hohe Temperatur erreicht ist und das Ventil schließt. Das Ventil kann von Hand geöffnet und nachfolgend können Gegenstände aus dem Behälter entnommen werden.

Die DE19908845 C1 offenbart einen Behälter zur Sterilisation von medizinischen Gegenständen, dessen Deckel durch eine ringförmige Dichtung verschlossen wird. Die ringförmige Dichtung enthält einen Hohlraum, dem zur Versiegelung die Luft entzogen werden kann. Trotz dieser aus dem Stand der Technik bekannten Lösungsansätze besteht weiterhin ein Bedarf an Behältern zum sterilen Verpacken von Gegenständen unter reduziertem Druck, die möglichst einfach aufgebaut sind und die sich vor allen Dingen schnell und unkompliziert unter Zerstörung des in dem Behälter vorliegenden Unterdrucks öffnen lassen.

### Darstellung der Erfindung

Hier setzt die Erfindung an. Es soll ein Behälter zum sterilen Verpacken von Gegenständen unter reduziertem Druck zur Verfügung gestellt werden, der einfach aufgebaut ist und der schnell und unkompliziert unter Zerstörung des in dem Behälter vorliegenden Unterdrucks geöffnet werden kann. Diese Aufgabe wird erfindungsgemäß durch den Behälter gemäß unabhängigem Anspruch 1 gelöst. Weitere vorteilhafte Aspekte, Details und Ausgestaltungen der Erfindung ergeben sich aus den abhängigen Ansprüchen, der Beschreibung sowie den Zeichnungen.

Die vorliegende Erfindung stellt einen Behälter zum sterilen Verpacken von Gegenständen unter reduziertem Druck zur Verfügung. Der Behälter umfasst ein Unterteil und ein Oberteil. Das Unterteil ist aus einem Boden und zumindest einer Unterteilseitenwand aufgebaut, wobei sich die zumindest eine Unterteilseitenwand vom Boden nach oben erstreckt und der Boden zusammen mit der zumindest einen Unterteilseitenwand einen Unterteilinnenraum mit einem offenen oberen Ende definiert. Das Oberteil ist aus einem Deckel und zumindest einer Oberteilseitenwand aufgebaut, wobei sich die zumindest eine Oberteilseitenwand vom Deckel nach unten erstreckt und der Deckel zusammen mit der zumindest einen Oberteilseitenwand einen Oberteilinnenraum mit einem offenen unteren Ende definiert. Der Behälter weist außerdem ein ringförmig ausgebildetes Dichtelement mit einer Dichtelementhauptebene auf, wobei das Dichtelement im Querschnitt senkrecht zur Dichtelementhauptebene H-förmig mit zwei Flanschen und einem Steg ausgebildet ist. Das Oberteil ist mit dem gesamten, dem offenen unteren Ende des Oberteils benachbarten Randbereich der Oberteilseitenwand und das Unterteil mit dem gesamten, dem offenen oberen Ende des Unterteils benachbarten Randbereich der Unterteilseitenwand zwischen die Flansche des Dichtelements einschiebbar ausgebildet. Das Dichtelement ist zum dichten Verschluss des Behälters ausgebildet, wobei der im zusammengebauten Zustand des Behälters an einer Außenseite des Behälters angeordnete Flansch des Dichtelements mit zumindest einer Sollrissstelle zum Zerstören des dichten Verschlusses des Behälters ausgestattet ist.

Nach Zusammenbau des Behälters aus Oberteil, Unterteil und Dichtelement befindet sich ein Flansch des H-förmigen Dichtelements im Behälterinnenraum und ein Flansch des H-förmigen Dichtelements ist an der Außenseite des Behälters angeordnet und liegt zum Teil an der Oberteilseitenwand und zum Teil an der Unterteilseitenwand an. Dieser, an der Außenseite des Behälters angeordnete und daher frei zugänglich und greifbare Flansch des Dichtelements ist erfindungsgemäß mit einer Sollrissstelle zum Zerstören des dichten Verschlusses des Behälters ausgestattet. Das Dichtelement stellt also neben seiner eigentlichen Funktion des dichten, insbesondere gasdichten, Verschließens des Behälters gleichzeitig eine Öffnungshilfe bereit, die es erlaubt, den Behälter ohne vorherige Betätigung eines Belüftungsventils zu öffnen. Der Öffnungsvorgang ist problemlos zu bewerkstelligen, da durch die Zerstörung der Sollrissstelle das Dichtelement seine dichtende Funktion verliert und somit Luft solange in den Behälter eindringt bis es zu einem vollständigen oder zumindest annähernd vollständigen Druckausgleich gekommen ist. Unterteil und Oberteil des Behälters können dann ohne besonderen Kraftaufwand voneinander getrennt werden.

Oberteil und Unterteil sind besonders bevorzugt einteilig ausgeführt. In diesem Fall ist keine Vormontage von Deckel und Oberteilseitenwand bzw. von Boden und Unterteilseitenwand erforderlich. Ebenfalls besonders bevorzugt sind Ausführungsformen, bei denen Oberteil und Unterteil jeweils aus einer einzigen, einstückig ausgeführten Seitenwand bestehen. In diesem Fall ist keine Vormontage mehrerer Seitenwände zur Bildung des Oberteilinnenraums bzw. des Unterteilinnenraums erforderlich.

Gemäß einer bevorzugten Ausführungsform besteht die Sollrissstelle zum Zerstören des dichten Verschlusses des Behälters aus zumindest einer, sich über die gesamte vertikale, senkrecht zur Dichtelementhauptebene orientierte Ausdehnung des im zusammengebauten Zustand des Behälters an einer Außenseite des Behälters angeordneten Flansches des Dichtelements erstreckende Perforierung. Eine Perforierung des außen am Behälter angeordneten Flansches des Dichtelements stellt eine besonders einfach herzustellende und problemlos zu betätigende Sollrissstelle dar. Es soll explizit darauf hingewiesen werden, dass gemäß der genannten Ausführungsform nur der außen am Behälter anliegende Flansch des Dichtelements perforiert ist, also nicht der Steg und nicht der sich im Behälterinnenraum befindende Flansch. Damit ist trotz der vorgesehenen Perforierung die Dichtungsfunktion des Dichtelements weiterhin gewährleistet.

Vorteilhafterweise ist die Sollrissstelle zum Zerstören des dichten Verschlusses des Behälters mit einer Kerbe ausgestattet, durch die ein leichteres Aufreißen des Dichtelements ermöglicht wird. Besonders einfach gestaltet sich dieser Vorgang, wenn eine solche Anrisskerbe mit der Perforierung des außen am Behälter angeordneten Flansches des Dichtelements in Wirkverbindung steht.

Gemäß einer weiteren, besonders bevorzugten Ausführungsform der vorliegenden Erfindung weist der im zusammengebauten Zustand des Behälters an der Außenseite des Behälters angeordnete Flansch des Dichtelements einen laschenartigen Fortsatz auf, wobei der laschenartige Fortsatz in Wirkverbindung mit der Sollrissstelle des Dichtelements steht. Besonders bevorzugt ist zudem eine mit der Sollrissstelle in Wirkverbindung stehende Anrisskerbe vorgesehen. Durch den laschenartigen Fortsatz und optional zusätzlich durch die Anrisskerbe wird ein besonders einfaches und problemloses Greifen des Dichtelements ermöglicht. Da der laschenartige Fortsatz in Wirkverbindung mit der Sollrissstelle und optional mit der Anrisskerbe des Dichtelements steht, kann durch ein einfaches Ziehen an dem laschenartigen Fortsatz die Sollrissstelle zerstört werden, wodurch das Dichtelement seine dichtende Funktion verliert und nachfolgend Unterteil und Oberteil des Behälters problemlos voneinander getrennt werden können. Diese Ausführungsform bringt ganz besondere Vorteile mit sich, wenn in dem Behälter medizinische Produkte, insbesondere chirurgische Instrumente, aufbewahrt werden. Im Operationssaal kommt es häufig zu Umständen, in denen der Person, die den Behälter öffnen soll, nur eine Hand für das Öffnen des Behälters zur Verfügung steht. Bei der vorliegenden Ausführungsform kann die an dem Dichtelement angeformte Lasche so gestaltet werden, dass mit ein wenig Übung die Sollrissstelle mit Hilfe der Lasche durch Greifen mit Daumen und Zeigefinger oder durch eine Schubbewegung des Daumens zerstört werden kann, während der Behälter zwischen zwei Finger derselben Hand eingeklemmt ist. Durch ein nachfolgendes Umkippen des Oberteils mit Hilfe des Daumens kann der Behälter insgesamt mit einer Hand geöffnet werden.

Oberteil und Unterteil des Behälters sollten aus dem gleichen Material gefertigt sein. Es können beispielsweise Kunststoffe als Wandungsmaterial verwendet werden. Besonders bevorzugt ist das Unterteil und das Oberteil aus Aluminium, einer Aluminiumlegierung oder aus Stahl gefertigt. Insbesondere der Werkstoff Aluminium ist gut formbar und zur Herstellung unterschiedlichster Behälterformen geeignet.

Bevorzugt weisen der Boden, die zumindest eine Unterteilseitenwand, der Deckel und die zumindest eine Oberteilseitenwand eine Wandungsstärke von 0,2 mm bis 1,5 mm, besonders bevorzugt von 0,5 mm bis 1,0 mm, insbesondere bevorzugt von rund 0,7 mm, auf. Die genannten Wandungsstärken lassen einerseits eine materialsparende Herstellung zu und verleihen andererseits dem Behälter eine ausreichende Formstabilität, die auch der nach dem Befüllen des Behälters unter Unterdruck zwischen dem Behälterinnenraum und der umgebenden Atmosphäre herrschenden Druckdifferenz widersteht. Besonders bevorzugt weisen der Boden, die zumindest eine Unterteilseitenwand, der Deckel und die zumindest eine Oberteilseitenwand die gleiche Wandungsstärke auf.

Sind Boden, Unterteilseitenwand, Deckel und Oberteilseitenwand aus dem gleichen Material gefertigt und weisen sie die gleiche Wandungsstärke auf, so ist auch bei einer eventuell auftretenden, von der zwischen dem Behälterinnenraum und der Umgebung herrschenden Druckdifferenz verursachten minimalen Verformung des Oberteils bzw. des Unterteils die Dichtigkeit des Behälters sichergestellt.

Bevorzugt ist die zumindest eine Unterteilseitenwand und/oder die zumindest eine Oberteilseitenwand mit zumindest einer Versteifungsrippe ausgestattet. Die Versteifungsrippe kann als Erhebung oder als Vertiefung der Unterteilseitenwand bzw. der Oberteilseitenwand ausgebildet sein und wird in die jeweilige Seitenwand bei konstanter Wandungsdicke eingeprägt. Die Versteifungsrippe kann eine Tiefe von rund 2 mm aufweisen, welche über den Verlauf der Versteifungsrippe hinweg konstant oder unterschiedlich sein kann. Insbesondere kann die Tiefe der Versteifungsrippe zum Rand der Wandungsvorder- bzw. -rückseite hin zu Null auslaufen. Durch die zumindest eine Versteifungsrippe wird die Formstabilität des Behälters weiter verbessert, sodass dieser auch einer nach dem Befüllen unter Unterdruck zwischen dem Behälterinnenraum und der umgebenden Atmosphäre herrschenden höheren Druckdifferenz widerstehen kann. Sowohl Unterteilseitenwand wie auch Oberteilseitenwand können vorteilhafterweise mit mehreren Versteifungsrippen ausgestattet sein.

Gemäß einer vorteilhaften Ausführungsform weist der Steg des Dichtelements eine vertikale Ausdehnung parallel zu den Flanschen von 1 mm bis 10 mm, bevorzugt von 1,5 mm bis 6 mm, besonders bevorzugt von rund 2 mm, auf. Der Steg ist vorteilhafterweise so dimensioniert, dass er durch die Randbereiche der Oberteilseitenwand und der Unterteilseitenwand beim Einschieben zwischen die Flansche des Dichtelements nicht durchstoßen werden kann und dass eine vorgesehene Sollrissstelle, wie beispielsweise eine Perforierung, nicht zum einem Verlust der Dichtfunktion des Dichtelements führt.

Gemäß einer weiteren vorteilhaften Ausführungsform weist der Steg des Dichtelements eine horizontale Ausdehnung senkrecht zu den Flanschen von 1 mm bis 8 mm, bevorzugt von 2 mm bis 5 mm, besonders bevorzugt von 3 mm, auf. Die horizontale Ausdehnung des Stegs des Dichtelements ist vorteilhafterweise auf die Wandungsstärke des Oberteils und des Unterteils abgestimmt.

Bevorzugt weisen die Flansche des Dichtelements eine vertikale, senkrecht zur Dichtelementhauptebene orientierte Ausdehnung von 5 mm bis 50 mm, bevorzugt von 10 mm bis 30 mm, besonders bevorzugt von rund 15 mm, auf. Die Flansche müssen sich von dem Steg ausgehend in beide Richtungen nach oben und nach unten über eine gewisse Distanz erstrecken, um die Dichtigkeit des Behälters gewährleisten zu können und auch um ein unbeabsichtigtes Öffnen des Behälters auszuschließen. Grundsätzlich kann die Ausdehnung der Flansche in vertikaler, senkrecht zur Dichtelementhauptebene orientierter Richtung an die Ausdehnung des Oberteils bzw. des Unterteils angepasst werden. Dabei wird man mit zunehmender Ausdehnung des Behälters auf eine zunehmende Ausdehnung der Flansche achten.

Das Dichtelement kann grundsätzlich aus sämtlichen für Dichtungen üblichen Materialien hergestellt werden. Bevorzugt ist das Dichtelement aus Kunststoff, Gummi oder Silikon gefertigt.

Es soll klar gestellt werden, dass Oberteil und Unterteil des Behälters grundsätzlich eine beliebige Form aufweisen können. So kann der Behälter insgesamt im horizontalen Querschnitt eine elliptische Form, eine rechteckige Form oder auch eine kreisrunde Form aufweisen. Es sind auch beliebig unregelmäßig geformte Behälter denkbar. Insbesondere bevorzugt weisen Oberteil und Unterteil einen identischen horizontalen Querschnitt auf. Eine unterschiedliche Form von Oberteil und Unterteil kann nur durch aufwändige Anpassung der Form des H-förmigen Dichtelements ausgeglichen werden.

Bevorzugt wird ein Behälter, bei dem die Form und Ausdehnung des Bodens und des Deckels im Wesentlichen gleich sind, bei dem die zumindest eine Oberteilseitenwand und der Deckel einen Winkel von rund 90° zueinander einschließen und bei dem die zumindest eine Unterteilseitenwand und der Boden einen Winkel von rund 90° zueinander einschließen.

Die vorliegende Erfindung umfasst auch die Verwendung eines der oben beschriebenen Behälter zum sterilen Verpacken von medizinischen Produkten unter reduziertem Druck. Wie bereits beschrieben schafft die vorliegende Erfindung die Möglichkeit, einen Behälter zum Aufbewahren medizinischer Produkte, insbesondere chirurgischer Instrumente, zur Verfügung zu stellen, der trotz der zwischen Behälterinnenraum und Außenumgebung herrschenden Druckdifferenz mit einer Hand geöffnet werden kann. Insbesondere im Operationssaal kommt es häufig zu Umständen, in denen einer Person zum Öffnen des Behälters nur eine Hand zur Verfügung steht. Dieser Umstand stellt bei der Verwendung eines der oben beschriebenen Behälter zum sterilen Verpacken von medizinischen Produkten unter reduziertem Druck einen ganz besonderen Vorteil dar. Besonders vorteilhaft sind Ausführungsformen, bei denen das Oberteil und das Unterteil so dimensioniert sind, dass der in dem Behälter aufbewahrte Gegenstand nach dem Öffnen des Behälters in einem der beiden Teile verbleibt, aus dem jeweiligen Teil herausragt und daher leicht entnommen werden kann.

Die vorliegende Erfindung umfasst auch ein Verfahren zum sterilen Verpacken von Gegenständen unter reduziertem Druck mit den Schritten
a) Bereitstellen eines der oben beschriebenen Behälter zum sterilen Verpacken von medizinischen Produkten unter reduziertem Druck in nicht zusammengebauten Zustand,
b) Einbringen des Unterteils, des Oberteils, des Dichtelements und des zumindest einen zu verpackenden Gegenstands in eine Umgebung unter reduziertem Druck,
c) Einbringen des zumindest einen zu verpackenden Gegenstands in den
Unterteilinnenraum oder den Oberteilinnenraum,
d1) Einschieben des dem offenen unteren Ende des Oberteils benachbarten Randbereichs der Oberteilseitenwand zwischen die Flansche des Dichtelements,
d2) Einschieben des dem offenen oberen Ende des Unterteils benachbarten Randbereichs der Unterteilseitenwand zwischen die Flansche des Dichtelements,
e) Erhöhung des Umgebungsdrucks auf Normaldruck,
wobei zumindest entweder Schritt d1) oder Schritt d2) nach Schritt b) durchgeführt wird, wobei zumindest entweder Schritt d1) oder Schritt d2) nach Schritt c) durchgeführt wird, wobei Schritt e) nach den Schritten c), d1) und d2) durchgeführt wird, und wobei das Einschieben in den Schritten d1) und d2) derart erfolgt, dass die Sollrissstelle des Dichtelements im zusammengebauten Zustand des Behälters an einer Außenseite des Behälters angeordnet ist.

Bei dem erfindungsgemäßen Verfahren kann das Oberteil oder das Unterteil bereits vor dem Einbringen in eine Umgebung unter reduziertem Druck zwischen die Flansche des Dichtelements eingeschoben werden. Es können aber auch alle Einzelteile separat in die Umgebung unter reduziertem Druck eingebracht werden und erst dort Ober- oder Unterteil mit dem Dichtelement verbunden werden. Eigentlich eine Selbstverständlichkeit stellt die Bedingung dar, dass das Einschieben des zweiten Teils und das damit verbundene Schließen des Behälters erst dann erfolgt, wenn der zu verpackende Gegenstand bereits in den Unterteilinnenraum oder den Oberteilinnenraum eingebracht wurde. Ebenso zwingend erforderlich ist es, dass das Einschieben von Oberteil und Unterteil zwischen die Flansche des Dichtelements derart erfolgt, dass die Sollrissstelle im zusammengebauten Zustand des Behälters an einer Außenseite des Behälters angeordnet ist und damit für den Benutzer zugänglich ist.

Weiterbildungen, Vorteile und Anwendungsmöglichkeiten der Erfindung ergeben sich auch aus der nachfolgenden Beschreibung von Ausführungsbeispielen und aus den Figuren.

### Kurze Beschreibung der Zeichnungen

Die Erfindung soll nachfolgend anhand eines Ausführungsbeispiels im Zusammenhang mit den Zeichnungen näher erläutert werden. Es zeigen
- Fig. 1: in schematischer Darstellung eine perspektivische Sicht auf einen erfindungsgemäßen Behälter;
- Fig. 2: in schematischer Darstellung eine Seitenansicht des Behälters gemäß Figur 1;
- Fig. 3: in schematischer Darstellung eine weitere Seitenansicht des Behälters gemäß Figur 1;
- Fig. 4: in schematischer Darstellung einen Ausschnitt der Figur 2 in vergrößerter Ansicht.

### Wege zur Ausführung der Erfindung

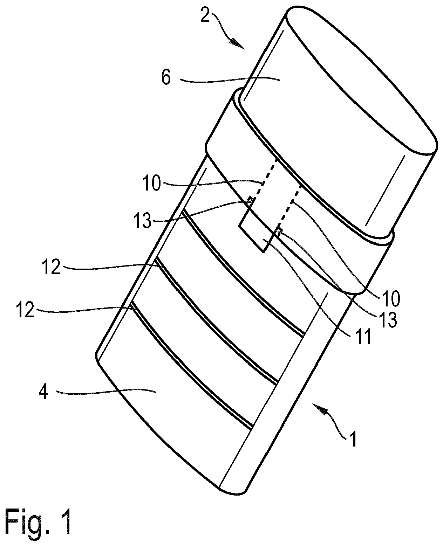

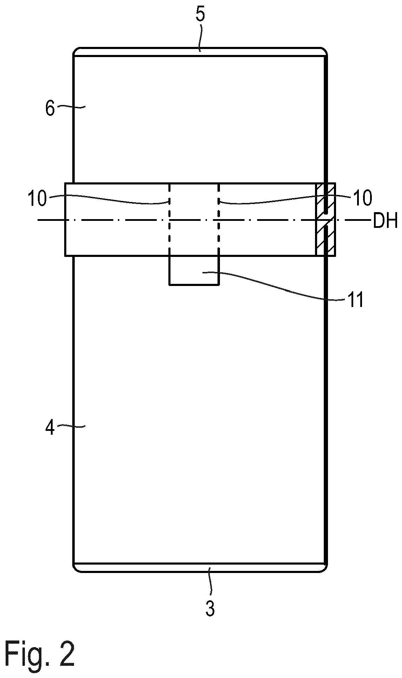

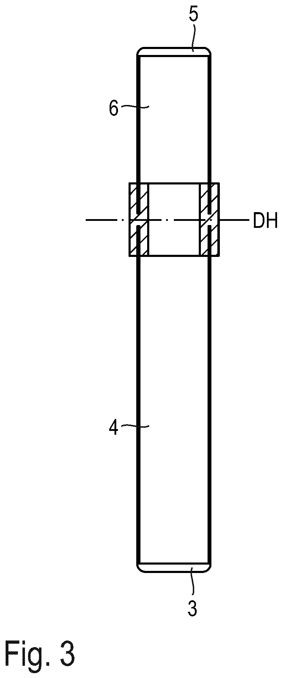

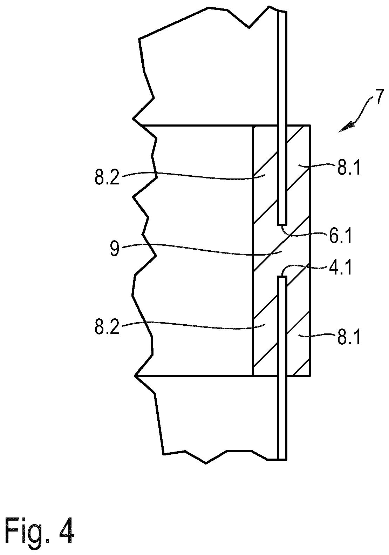

Die Figuren 1 bis 4 zeigen in schematischer Darstellung verschiedene Ansichten eines erfindungsgemäßen Behälters zum sterilen Verpacken von Gegenständen unter reduziertem Druck.

Der Behälter umfasst ein Unterteil 1 und ein Oberteil 2. Das Unterteil 1 ist einstückig ausgebildet und aus einem Boden 3 und einer Unterteilseitenwand 4 aufgebaut, wobei sich die Unterteilseitenwand 4 vom Boden 3 senkrecht nach oben erstreckt und der Boden 3 zusammen mit der einen Unterteilseitenwand 4 einen Unterteilinnenraum mit einem offenen oberen Ende definiert. Das Oberteil 2 ist ebenfalls einstückig ausgebildet und aus einem Deckel 5 und einer Oberteilseitenwand 6 aufgebaut, wobei sich die Oberteilseitenwand 6 vom Deckel 5 senkrecht nach unten erstreckt und der Deckel 5 zusammen mit der zumindest einen Oberteilseitenwand 6 einen Oberteilinnenraum mit einem offenen unteren Ende definiert.

Oberteil 2 und Unterteil 1 des Behälters sind aus Aluminium gefertigt. Der Boden 3, die Unterteilseitenwand 4, der Deckel 5 und die Oberteilseitenwand 6 weisen eine Wandungsstärke von rund 0,7 mm, auf. Die Unterteilseitenwand 4 ist mit drei Versteifungsrippen 12 ausgestattet (nur in Figur 1 gezeigt). Die genannte Wandungsstärke und die Versteifungsrippen ermöglichen zum einen eine materialsparende Herstellung und verleihen daneben dem Behälter eine ausreichende Formstabilität, die auch der nach dem Befüllen des Behälters unter Unterdruck zwischen Behälterinnenraum und umgebender Atmosphäre herrschenden Druckdifferenz problemlos widersteht.

Der Behälter weist außerdem ein ringförmig ausgebildetes Dichtelement 7 mit einer Dichtelementhauptebene DH auf, wobei das Dichtelement 7 im Querschnitt senkrecht zur Dichtelementhauptebene DH H-förmig mit zwei Flanschen 8.1, 8.2 und einem Steg 9 ausgebildet ist. Das Oberteil 2 ist mit dem gesamten, dem offenen unteren Ende des Oberteils benachbarten Randbereich 6.1 der Oberteilseitenwand 6 und das Unterteil 3 mit dem gesamten, dem offenen oberen Ende des Unterteils benachbarten Randbereich 4.1 der Unterteilseitenwand 4 zwischen die Flansche 8.1, 8.2 des Dichtelements 7 eingeschoben. Das aus Silikon bestehende Dichtelement 7 verschließt den Behälter dicht. Der an der Außenseite des Behälters angeordnete Flansch 8.1 des Dichtelements 7 ist mit zwei Sollrissstellen 10 und zwei, mit jeweils einer Sollrissstelle 10 in Wirkverbindung stehenden Anrisskerben 13 zum Zerstören des dichten Verschlusses des Behälters ausgestattet. Die Sollrissstellen 10 sind in Form einer Perforierung des Flansches 8.1 ausgebildet.

Der an der Außenseite des Behälters angeordnete Flansch 8.1 des Dichtelements 7 weist einen laschenartigen Fortsatz 11 auf, wobei der laschenartige Fortsatz 11 in Wirkverbindung mit den beiden Sollrissstellen 10 des Dichtelements 7 steht. Durch den laschenartigen Fortsatz 11 wird ein besonders einfaches und problemloses Greifen des Dichtelements ermöglicht. Da der laschenartige Fortsatz 11 in Wirkverbindung mit den Sollrissstellen 10 des Dichtelements 7 steht, können durch ein einfaches Ziehen an dem laschenartigen Fortsatz 11 die Sollrissstellen 10 zerstört werden, wodurch das Dichtelement 7 seine dichtende Funktion verliert und nachfolgend Unterteil 1 und Oberteil 2 des Behälters problemlos voneinander getrennt werden können. Die an dem Dichtelement 7 angeformte Lasche 11 ist so gestaltet, dass die Sollrissstellen 10 mit Hilfe der Lasche 11 durch Greifen mit Daumen und Zeigefinger oder durch eine Schubbewegung des Daumens zerstört werden können, während der Behälter zwischen zwei Fingern derselben Hand eingeklemmt ist. Durch das nachfolgende Umkippen des Oberteils mit Hilfe des Daumens kann der Behälter insgesamt mit einer Hand geöffnet werden.

Der Steg 9 des Dichtelements 7 weist eine vertikale Ausdehnung parallel zu den Flanschen 8.1, 8.2 von rund 4 mm auf. Der Steg ist so dimensioniert, dass er durch die Randbereiche 6.1, 4.1 der Oberteilseitenwand 6 und der Unterteilseitenwand 4 beim Einschieben zwischen die Flansche 8.1, 8.2 des Dichtelements 7 nicht durchstoßen wird. Gleichzeitig können die Perforierungen 10 nicht zu einem Verlust der Dichtfunktion des Dichtelements 7 führen.

Die horizontale Ausdehnung des Stegs 9 des Dichtelements 7 senkrecht zu den Flanschen 8.1, 8.2 beträgt 3 mm und ist damit gut auf die Wandungsstärke der Randbereiche 6.1, 4.1 der Oberteilseitenwand 6 und der Unterteilseitenwand 4, welche jeweils rund 0,7 mm beträgt, abgestimmt.

Die Flansche 8.1, 8.2 des Dichtelements 7 weisen eine vertikale, senkrecht zur Dichtelementhauptebene DH orientierte Ausdehnung von rund 20 mm auf. Die Flansche erstrecken sich von dem Steg ausgehend in beide Richtungen nach oben und nach unten über eine Distanz von 8 mm, wodurch die Dichtigkeit des Behälters gewährleistet ist und auch ein unbeabsichtigtes Öffnen des Behälters ausgeschlossen ist. Die Ausdehnung der Flansche 8.1, 8.2 in vertikaler, senkrecht zur Dichtelementhauptebene DH orientierter Richtung ist damit gut an die Ausdehnung des Oberteils 2 von rund 45 mm bzw. des Unterteils 1 von rund 95 mm angepasst.

### Bezugszeichenliste

- 1: Unterteil
- 2: Oberteil
- 3: Boden
- 4: Unterteilseitenwand
- 4.1: Randbereich der Unterteilseitenwand
- 5: Deckel
- 6: Oberteilseitenwand
- 6.1: Randbereich der Oberteilseitenwand
- 7: Dichtelement
- 8.1, 8.2: Flansche des Dichtelements
- 9: Steg des Dichtelements
- 10: Sollrissstelle
- 11: laschenartigen Fortsatz
- 12: Versteifungsrippen
- 13: Anrisskerbe

- DH: Dichtelementhauptebene

## Patentansprüche

1. Behälter zum sterilen Verpacken von Gegenständen unter reduziertem Druck, wobei der Behälter ein Unterteil (1) und ein Oberteil (2) umfasst, wobei das Unterteil (1) aus einem Boden (3) und zumindest einer Unterteilseitenwand (4) aufgebaut ist, wobei sich die zumindest eine Unterteilseitenwand (4) vom Boden (3) nach oben erstreckt und der Boden (3) zusammen mit der zumindest einen Unterteilseitenwand (4) einen Unterteilinnenraum mit einem offenen oberen Ende definiert, wobei das Oberteil (2) aus einem Deckel (5) und zumindest einer Oberteilseitenwand (6) aufgebaut ist, wobei sich die zumindest eine Oberteilseitenwand (6) vom Deckel (5) nach unten erstreckt und der Deckel (5) zusammen mit der zumindest einen Oberteilseitenwand (6) einen Oberteilinnenraum mit einem offenen unteren Ende definiert, wobei der Behälter ein ringförmig ausgebildetes Dichtelement (7) mit einer Dichtelementhauptebene (DH) aufweist, **dadurch gekennzeichnet, dass** das Dichtelement (7) im Querschnitt senkrecht zur Dichtelementhauptebene (DH) H-förmig mit zwei Flanschen (8.1, 8.2) und einem Steg (9) ausgebildet ist, wobei das Oberteil (2) mit dem gesamten, dem offenen unteren Ende des Oberteils benachbarten Randbereich (6.1) der Oberteilseitenwand (6) und das Unterteil (3) mit dem gesamten, dem offenen oberen Ende des Unterteils benachbarten Randbereich (4.1) der Unterteilseitenwand (4) zwischen die Flansche (8.1, 8.2) des Dichtelements (7) einschiebbar ausgebildet sind, wobei das Dichtelement (7) zum dichten Verschluss des Behälters ausgebildet ist, wobei der im zusammengebauten Zustand des Behälters an einer Außenseite des Behälters angeordnete Flansch (8.1) des Dichtelements (7) mit zumindest einer Sollrissstelle (10) zum Zerstören des dichten Verschlusses des Behälters ausgestattet ist.

2. Behälter nach Anspruch 1, **dadurch gekennzeichnet, dass** die Sollrissstelle (10) zum Zerstören des dichten Verschlusses des Behälters aus zumindest einer, sich über die gesamte vertikale, senkrecht zur Dichtelementhauptebene (DH) orientierte Ausdehnung des im zusammengebauten Zustand des Behälters an einer Außenseite des Behälters angeordneten Flansches (8.1) des Dichtelements (7) erstreckende Perforierung besteht.

3. Behälter nach Anspruch 1 oder 2, **dadurch gekennzeichnet, dass** der im zusammengebauten Zustand des Behälters an einer Außenseite des Behälters angeordnete Flansch (8.1) des Dichtelements (7) einen laschenartigen Fortsatz (11) aufweist, wobei der laschenartige Fortsatz (11) in Wirkverbindung mit der Sollrissstelle (10) des Dichtelements (7) steht.

4. Behälter nach einem der Ansprüche 1 bis 3, **dadurch gekennzeichnet, dass** zumindest eine, mit einer Sollrissstelle (10) in Wirkverbindung stehende Anrisskerbe vorgesehen ist.

5. Behälter nach einem der Ansprüche 1 bis 4, **dadurch gekennzeichnet, dass** das Unterteil (1) und/oder das Oberteil (2) aus Aluminium, einer Aluminiumlegierung oder aus Stahl besteht.

6. Behälter nach einem der Ansprüche 1 bis 5, **dadurch gekennzeichnet, dass** der Boden (3), die zumindest eine Unterteilseitenwand (4), der Deckel (5) und die zumindest eine Oberteilseitenwand (6) eine Wandungsstärke von 0,2 mm bis 1,5 mm, bevorzugt von 0,5 mm bis 1,0 mm, besonders bevorzugt von rund 0,7 mm, aufweisen.

7. Behälter nach einem der Ansprüche 1 bis 6, **dadurch gekennzeichnet, dass** die zumindest eine Unterteilseitenwand (4) und/oder die zumindest eine Oberteilseitenwand (6) mit zumindest einer Versteifungsrippe (12) ausgestattet ist.

8. Behälter nach einem der Ansprüche 1 bis 7, **dadurch gekennzeichnet, dass** der Steg (9) des Dichtelements (7) eine vertikale Ausdehnung parallel zu den Flanschen (8.1, 8.2) von 1 mm bis 10 mm, bevorzugt von 1,5 mm bis 6 mm, besonders bevorzugt von rund 2 mm, aufweist.

9. Behälter nach einem der Ansprüche 1 bis 8, **dadurch gekennzeichnet, dass** der Steg (9) des Dichtelements (7) eine horizontale Ausdehnung senkrecht zu den Flanschen (8.1, 8.2) von 1 mm bis 8 mm, bevorzugt von 2 mm bis 5 mm, besonders bevorzugt von 3 mm, aufweist.

10. Behälter nach einem der Ansprüche 1 bis 9, **dadurch gekennzeichnet, dass** die Flansche (8.1, 8.2) des Dichtelements (7) eine vertikale, senkrecht zur Dichtelementhauptebene (DH) orientierte Ausdehnung von 5 mm bis 50 mm, bevorzugt von 10 mm bis 30 mm, besonders bevorzugt von rund 15 mm, aufweisen.

11. Behälter nach einem der Ansprüche 1 bis 10, **dadurch gekennzeichnet, dass** das Dichtelement (7) aus Kunststoff, Gummi oder Silikon besteht.

12. Behälter nach einem der Ansprüche 1 bis 11, **dadurch gekennzeichnet, dass**
- Form und Ausdehnung des Bodens (3) und des Deckels (5) im Wesentlichen gleich sind,
- die zumindest eine Oberteilseitenwand (6) und der Deckel (5) einen Winkel von rund 90° zueinander einschließen und
- die zumindest eine Unterteilseitenwand (4) und der Boden (3) einen Winkel von rund 90° zueinander einschließen.

13. Verwendung eines Behälters nach einem der Ansprüche 1 bis 12 zum sterilen Verpacken von medizinischen Produkten unter reduziertem Druck.

14. Verfahren zum sterilen Verpacken von Gegenständen unter reduziertem Druck mit den Schritten
a) Bereitstellen eines Behälters gemäß einem der Ansprüche 1 bis 12 in nicht zusammengebauten Zustand,
b) Einbringen des Unterteils (1), des Oberteils (2), des Dichtelements (7) und des zumindest einen zu verpackenden Gegenstands in eine Umgebung unter reduziertem Druck,
c) Einbringen des zumindest einen zu verpackenden Gegenstands in den Unterteilinnenraum oder den Oberteilinnenraum,
d1) Einschieben des dem offenen unteren Ende des Oberteils benachbarten Randbereichs (6.1) der Oberteilseitenwand (6) zwischen die Flansche (8.1, 8.2) des Dichtelements (7),
d2) Einschieben des dem offenen oberen Ende des Unterteils benachbarten Randbereichs (4.1) der Unterteilseitenwand (4) zwischen die Flansche (8.1, 8.2) des Dichtelements (7),
e) Erhöhung des Umgebungsdrucks auf Normaldruck,
wobei zumindest entweder Schritt d1) oder Schritt d2) nach Schritt b) durchgeführt wird,
wobei zumindest entweder Schritt d1) oder Schritt d2) nach Schritt c) durchgeführt wird,
wobei Schritt e) nach den Schritten c), d1) und d2) durchgeführt wird, und
wobei das Einschieben in den Schritten d1) und d2) derart erfolgt, dass die Sollrissstelle des Dichtelements (7) im zusammengebauten Zustand des Behälters an einer Außenseite des Behälters angeordnet ist.

## Claims

1. A container for sterile packaging objects under reduced pressure, the container comprising a lower part (1) and an upper part (2), wherein the lower part (1) is constructed from a bottom (3) and at least one lower-part side wall (4), wherein the at least one lower-part side wall (4) extends upwards from the bottom (3) and the bottom (3) together with the at least one lower-part side wall (4) defines a lower-part interior with an open upper end, wherein the upper part (2) is constructed from a cover (5) and at least one upper-part side wall (6), wherein the at least one upper-part side wall (6) extends downwards from the cover (5) and the cover (5) together with the at least one upper-part side wall (6) defines an upper-part interior with an open lower end, wherein the container comprises an annular sealing element (7) with a sealing-element main plane (DH), **characterized in that** the sealing element (7) is designed H-shaped in cross-section perpendicular to the sealing-element main plane (DH) with two flanges (8.1, 8.2) and a web (9), wherein the upper part (2) is designed such that it can be slid in between the flanges (8.1, 8.2) of the sealing element (7) with the entire edge region (6.1) of the upper-part side wall (6) adjacent to the open lower end of the upper part and wherein the lower part (3) is designed such that it can be slid in between the flanges (8.1, 8.2) of the sealing element (7) with the entire edge region (4.1) of the lower-part side wall (4) adjacent to the open upper end of the lower part, wherein the sealing element (7) is designed to tightly close the container, wherein the flange (8.1) of the sealing element (7) which is arranged on an outside of the container in the assembled state of the container is equipped with at least one predetermined tearing feature (10) for destroying the tight closure of the container.

2. Container according to claim 1, **characterized in that** the predetermined tearing feature (10) for destroying the tight closure of the container consists of at least one perforation extending over the entire vertical extent of the flange (8.1) of the sealing element (7) arranged on an outside of the container in the assembled state of the container, said vertical extent being oriented perpendicular to the sealing-element main plane (DH).

3. Container according to claim 1 or 2, **characterized in that** the flange (8.1) of the sealing element (7) arranged on an outside of the container in the assembled state of the container has a flap-like extension (11), wherein the flap-like extension (11) is operatively connected to the predetermined tearing feature (10) of the sealing element (7).

4. Container according to any one of claims 1 to 3, **characterized in that** at least one crack initiation notch is provided which is operatively connected to a predetermined tearing feature (10).

5. Container according to any one of claims 1 to 4, **characterized in that** the lower part (1) and/or the upper part (2) are/is made of aluminum, an aluminum alloy or steel.

6. Container according to any one of claims 1 to 5, **characterized in that** the bottom (3), the at least one lower-part side wall (4), the cover (5) and the at least one upper-part side wall (6) have a wall thickness of 0.2 mm to 1.5 mm, preferably of 0.5 mm to 1.0 mm, particularly preferably of approximately 0.7 mm.

7. Container according to any one of claims 1 to 6, **characterized in that** the at least one lower-part side wall (4) and/or the at least one upper-part side wall (6) are/is equipped with at least one stiffening rib (12).

8. Container according to any one of claims 1 to 7, **characterized in that** the web (9) of the sealing element (7) has a vertical extent parallel to the flanges (8.1, 8.2) of 1 mm to 10 mm, preferably of 1.5 mm to 6 mm, particularly preferably of approximately 2 mm.

9. Container according to any one of claims 1 to 8, **characterized in that** the web (9) of the sealing element (7) has a horizontal extent perpendicular to the flanges (8.1, 8.2) of 1 mm to 8 mm, preferably of 2 mm to 5 mm, particularly preferably of 3 mm.

10. Container according to any one of claims 1 to 9, **characterized in that** the flanges (8.1, 8.2) of the sealing element (7) have a vertical extent oriented perpendicular to the sealing-element main plane (DH) of 5 mm to 50 mm, preferably of 10 mm to 30 mm, particularly preferably of approximately 15 mm.

11. Container according to any one of claims 1 to 10, **characterized in that** the sealing element (7) is made of plastics, rubber or silicone.

12. Container according to any of claims 1 to 11, **characterized in that**
- the shape and extent of the bottom (3) and the cover (5) are substantially the same,
- the at least one upper-part side wall (6) and the cover (5) enclose an angle of approximately 90° to one another, and
- the at least one lower-part side wall (4) and the bottom (3) enclose an angle of approximately 90° to one another.

13. Use of a container according to any one of claims 1 to 12 for sterile packaging of medical products under reduced pressure.

14. A method for sterile packaging of objects under reduced pressure, comprising the steps of
a) providing a container according to any one of claims 1 to 12 in an unassembled state,
b) introducing the lower part (1), the upper part (2), the sealing element (7) and the at least one object to be packaged into an environment under reduced pressure,
c) introducing the at least one object to be packaged into the lower-part interior or the upper-part interior,
d1) sliding in the edge region (6.1) of the upper-part side wall (6) adjacent to the open lower end of the upper part between the flanges (8.1, 8.2) of the sealing element (7),
d2) sliding in the edge region (4.1) of the lower-part side wall (4) adjacent to the open upper end of the lower part between the flanges (8.1, 8.2) of the sealing element (7),
(e) increasing the ambient pressure to normal pressure,
wherein at least either step d1) or step d2) is performed after step b),
wherein at least either step d1) or step d2) is performed after step c),
wherein step e) is performed after steps c), d1) and d2), and
wherein the sliding-in in steps d1) and d2) is carried out in such a manner that the predetermined tearing feature of the sealing element (7) is arranged on an outside of the container in the assembled state of the container.

## Revendications

1. Conteneur pour l'emballage stérile d'objets sous pression réduite, le conteneur comprenant une partie inférieure (1) et une partie supérieure (2), la partie inférieure (1) étant constituée d'un fond (3) et au moins d'une paroi latérale de partie inférieure (4), ladite au moins une paroi latérale de partie inférieure (4) s'étendant du fond (3) vers le haut et le fond (3) définissant avec ladite au moins une paroi latérale de partie inférieure (4) un espace intérieur de partie inférieure avec une extrémité supérieure ouverte, la partie supérieure (2) étant constituée d'un couvercle (5) et d'au moins une paroi latérale de partie supérieure (6), ladite au moins une paroi latérale de partie supérieure (6) s'étendant du couvercle (5) vers le bas et le couvercle (5) définissant avec ladite au moins une paroi latérale de partie supérieure (6) un espace intérieur de partie supérieure avec une extrémité inférieure ouverte, le conteneur comportant un élément d'étanchéité (7) constitué de forme annulaire avec un plan principal d'élément d'étanchéité (DH), **caractérisé en ce que** l'élément d'étanchéité (7) est constitué en section transversale perpendiculairement au plan principal d'élément d'étanchéité (DH) en forme de H avec deux brides (8.1, 8.2) et une membrure (9), la partie supérieure (2) avec toute la zone de bordure (6.1) de la paroi latérale de partie supérieure (6), voisine de l'extrémité inférieure ouverte de la partie supérieure et la partie inférieure (3) avec toute la zone de bordure (4.1) de la paroi latérale de partie inférieure (4), voisine de l'extrémité supérieure ouverte de la partie inférieure, sont constituées pouvant être insérées entre les brides (8.1, 8.2) de l'élément d'étanchéité (7), l'élément d'étanchéité (7) étant constitué pour la fermeture étanche du conteneur, la bride (8.1) de l'élément d'étanchéité (7) disposée à l'état assemblé du conteneur sur une face extérieure du conteneur est dotée d'au moins un point de déchirure théorique (10) pour détruire la fermeture étanche du conteneur.

2. Conteneur selon la revendication 1, **caractérisé en ce que** le point de déchirure théorique (10) pour détruire la fermeture étanche du conteneur est composé au moins d'une perforation s'étendant sur toute l'extension verticale orientée perpendiculairement au plan principal de l'élément d'étanchéité (DH) de la bride (8.1) de l'élément d'étanchéité (7) disposée à l'état assemblé du conteneur sur une face extérieure du conteneur.

3. Conteneur selon la revendication 1 ou 2, **caractérisé en ce que** la bride (8.1) de l'élément d'étanchéité (7) disposée à l'état assemblé du conteneur sur une face extérieure du conteneur comporte une prolongation de type languette (11), la prolongation de type languette (11) étant en liaison active avec le point de déchirure théorique (10) de l'élément d'étanchéité (7).

4. Conteneur selon l'une quelconque des revendications 1 à 3, **caractérisé en ce que** au moins une entaille d'amorce de fissuration en liaison active avec un point de déchirure théorique (10) est prévue.

5. Conteneur selon l'une quelconque des revendications 1 à 4, **caractérisé en ce que** la partie inférieure (1) et/ou la partie supérieure (2) est faite en aluminium, dans un alliage d'aluminium ou en acier.

6. Conteneur selon l'une quelconque des revendications 1 à 5, **caractérisé en ce que** le fond (3), ladite au moins une paroi latérale de partie inférieure (4), le couvercle (5) et ladite au moins une paroi latérale de partie supérieure (6) comportent une épaisseur de paroi de 0,2 mm à 1,5 mm, de préférence de 0,5 mm à 1,0 mm, de façon particulièrement préférée d'environ 0,7 mm.

7. Conteneur selon l'une quelconque des revendications 1 à 6, **caractérisé en ce que** ladite au moins une paroi latérale de partie inférieure (4) et/ou ladite au moins une paroi latérale de partie supérieure (6) est dotée d'au moins une nervure raidisseuse (12).

8. Conteneur selon l'une quelconque des revendications 1 à 7, **caractérisé en ce que** la membrure (9) de l'élément d'étanchéité (7) comporte une extension verticale parallèle aux brides (8.1, 8.2) de 1 mm à 10 mm, de préférence de 1,5 mm à 6 mm, de façon particulièrement préférée d'environ 2 mm.

9. Conteneur selon l'une quelconque des revendications 1 à 8, **caractérisé en ce que** la membrure (9) de l'élément d'étanchéité (7) comporte une extension horizontale perpendiculaire aux brides (8.1, 8.2) de 1 mm à 8 mm, de préférence de 2 mm à 5 mm, de façon particulièrement préférée de 3 mm.

10. Conteneur selon l'une quelconque des revendications 1 à 9, **caractérisé en ce que** les brides (8.1, 8.2) de l'élément d'étanchéité (7) comportent une extension verticale, orientée perpendiculairement au plan principal d'élément d'étanchéité (DH) de 5 mm à 50 mm, de préférence de 10 mm à 30 mm, de façon particulièrement préférée d'environ 15 mm.

11. Conteneur selon l'une quelconque des revendications 1 à 10, **caractérisé en ce que** l'élément d'étanchéité (7) est en matière plastique, en caoutchouc ou en silicone.

12. Conteneur selon l'une quelconque des revendications 1 à 11, **caractérisé en ce que**
- la forme et l'extension du fond (3) et du couvercle (5) sont pour l'essentiel identiques,
- ladite au moins une paroi latérale de partie supérieure (6) et le couvercle (5) forment un angle d'environ 90° l'un par rapport à l'autre, et
- ladite au moins une paroi latérale de partie inférieure (4) et le fond (3) forment un angle d'environ 90° l'un par rapport à l'autre.

13. Utilisation d'un conteneur selon l'une quelconque des revendications 1 à 12 pour l'emballage stérile de produits médicaux sous pression réduite.

14. Procédé d'emballage stérile d'objets sous pression réduite comportant les étapes :
a) Fourniture d'un conteneur selon l'une quelconque des revendications 1 à 12 à l'état non assemblé,
b) mise en place de la partie inférieure (1), de la partie supérieure (2), de l'élément d'étanchéité (7) et d'au moins un objet à emballer dans un environnement sous pression réduite,
c) mise en place d'au moins un objet à emballer dans l'espace intérieur de partie inférieure ou l'espace intérieur de partie supérieure,
d1) insertion de la zone de bordure (6.1) de la paroi latérale de partie inférieure (6), voisine de l'extrémité inférieure ouverte de la partie supérieure, entre les brides (8.1, 8.2) de l'élément d'étanchéité (7),
d2) insertion de la zone de bordure (4.1) de la paroi latérale de partie inférieure (4), voisine de l'extrémité supérieure ouverte de la partie inférieure entre les brides (8.1, 8.2) de l'élément d'étanchéité (7),
e) augmentation de la pression environnante à la pression normale,
au moins soit l'étape d1), soit l'étape d2) étant effectuée après l'étape b),
au moins soit l'étape d1), soit l'étape d2) étant effectuée après l'étape c), l'étape e) étant effectuée après les étapes c), d1) et d2), et
l'insertion dans les étapes d1) et d2) ayant lieu de telle sorte que le point de déchirure théorique de l'élément d'étanchéité (7) est disposé à l'état assemblé du conteneur sur une face extérieure du conteneur.
